(19) Europäisches Patentamt
European Patent Office
Office européen des brevets

(11) Publication number : 0 496 824 B1

# (12) EUROPEAN PATENT SPECIFICATION

(45) Date of publication of patent specification : **29.12.93 Bulletin 93/52**

(51) Int. Cl.$^5$ : **A61K 9/127**

(21) Application number : **90916921.1**

(22) Date of filing : **26.10.90**

(86) International application number : **PCT/US90/06183**

(87) International publication number : **WO 91/06285 16.05.91 Gazette 91/11**

## (54) RECONSTITUTION METHOD.

(30) Priority : **27.10.89 US 428343**

(43) Date of publication of application : **05.08.92 Bulletin 92/32**

(45) Publication of the grant of the patent : **29.12.93 Bulletin 93/52**

(84) Designated Contracting States : **AT BE CH DE DK ES FR GB GR IT LI LU NL SE**

(56) References cited :
**WO-A-88/09168**
**Acta Pharmaceutica Technologica, vol. 34, no. 3, September 1988, (Stuttgart, DE), Y,özer et al.: " Influence of freezing and freeze-drying on the stability of liposomes dispersed in aqueous media", see the whole article**

(73) Proprietor : **VESTAR, INC.**
**650 Cliffside Drive**
**San Dimas, CA 91773 (US)**

(72) Inventor : **ELEY, Crispin, G., S.**
**650 W. Hermosa Drive**
**Fullerton, CA 92635 (US)**

(74) Representative : **Brown, David Leslie et al**
**Page Hargrave Temple Gate House Temple Gate**
**Bristol BS1 6PL (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 496 824 B1

## Description

This invention relates generally to pharmaceutical sciences and more particularly to the commercial production of liposomes and other lipid bilayer vesicles.

Lipid bilayer vesicles are closed microscopic vesicles which are formed principally from individual molecules having a polar (hydrophilic) and a non-polar (lipophilic) portion, although cholesterol and other sterols may be included if desired. The hydrophilic groups can be phosphato, glycerylphosphato, carboxy, sulfato, amino, hydroxy, choline or other polar groups. Examples of non-polar groups are saturated or unsaturated hydrocarbons such as alkyl, alkenyl or other lipid groups. Liposomes are a subset of these bilayer vesicles and are comprised principally of phospholipid molecules. Upon exposure to water, these molecules form a bilayer membrane with the lipid ends of the molecules in each layer associated in the center of the membrane and the opposing polar ends forming the respective inner and outer surface of the bilayer membrane. Thus, each side of the membrane presents a hydrophilic surface while the interior of the membrane comprises a lipophilic medium. These membranes, in the presence of excess water, can initially be arranged in a system of concentric closed membranes, in a manner which is not dissimilar to the layers of an onion, around an internal aqueous space. Each of the membranes is an unbroken, bilayer sheet of lipid molecules. With additional energy such as homogenization, sonication or extrusion, these multilamellar vesicles (MLVs) can form small unilamellar vesicles (SUVs). The technical aspects of liposome formation are well known in the art, as is the fact that liposomes are advantageous for encapsulating biologically active substances.

While liposomes offer significant advantages, particularly as delivery vehicles for pharmaceuticals, it is also known that liposomes and their drug contents may be relatively unstable in aqueous dispersion. Accordingly, workers in the art have attempted to increase the relatively short storage life of certain liposomal products by dehydrating the dispersion, for example, by lyophilizing (freeze drying) the aqueous dispersion to form a stable powder which can be stored for a long period and from which, with an aqueous medium, a liposome dispersion can be reconstituted.

Early attempts to dehydrate lipid bilayer vesicles such as liposomes were not successful, due in part to the fact that lipid bilayer membranes are most stable in the presence of the aqueous phase which maintains the molecular orientation. However, a significant improvement is provided through the use of various hydrophilic membrane stabilizing agents, *e.g.*, carbohydrates such as sucrose, which are thought to support the individual lipid particles during drying. See, for example, Racker (1972) *J. Membrane BioL* 10, 221-235; and U.S. Patent 4,229,360 to Schneider, *et al.*

Notwithstanding the use of hydrophilic membrane stabilizing agents, which may provide a visually acceptable liposome powder, traditional methods for the reconstitution of the dehydrated or lyophilized powder, *ie.,* the addition of an aqueous phase at controlled room temperature (15° to 30° as defined by the U.S. Pharmacopeia) to form the final liposome dispersion, often yields unpredictable results. In particular, the powder often forms extremely large liposomes or lipid globules rather than reforming liposomes which have comparable size, size distribution and integrity to that of the initial liposome dispersion. Accordingly, it has been a desideratum to provide a reliable method for the reconstitution of dehydrated lipid bilayer vesicle powders, and particularly lyophilized powders, into usable dispersions with defined and predictable size distributions.

According to the invention, a method for the reconstitution of dried lipid bilayer vesicles is provided, which comprises adding a quantity of a liquid aqueous phase having a temperature of less than about 15° to the dried particles to form an aqueous particulate dispersion. Preferably, the bilayer vesicles are liposomes and most preferably the liposomes are small unilamellar vesicles which are reconstituted at a temperature of from about 2° to 8°. As used herein, the term lipid bilayer vesicle describes a microscopic cell or sac defined by one or more lipid bilayer membranes, and includes liposomes. The term dried vesicles or dried liposomes refers to the product formed by the evaporation of water from a dispersion of the respective lipid vesicles, and the term reconstitution refers to the formation of a dispersion of the vesicles or liposomes in a liquid phase in a manner which substantially controls the size and size distribution of the vesicles to maintain these parameters at values close to those in the initial dispersion prior to dehydration. All temperatures herein are expressed as degrees Celsius.

While I do not wish to be bound by any particular theory, it appears that traditional room temperature (15° to 30°) hydration of dried vesicle powder dissolves the supporting membrane stabilizing agents prior to sufficient intercalation of the water to stabilize the individual lipid molecules in the initial bilayer form.

In another aspect of the invention, the parameters of the reconstitution temperature, the amount and form of one or more hydrophilic membrane stabilizing agents, and other factors, such as buffers, are controlled to provide optimal reconstitution of vesicles under a wide range of conditions.

## Brief Description of the Drawing

The Figure is a graph of the 650 - 750nm light scattering index described herein plotted against the temperature of the rehydrating aqueous phase (water for injection, USP) for various lots of dehydrated liposomal amphotericin B powder.

## Detailed Description

As mentioned above, methods for the formation of the initial liposome dispersion are well known in the art, and are not part of the method of the invention. Similarly, particular methods for the dehydration of the initial liposome dispersion are known, including the use of the hydrophilic membrane stabilizing agents referenced above. Accordingly only a general description of liposome production and drying will be mentioned herein, with primary emphasis being placed on the invention, that is, the rehydration of dried bilayer particles such as liposomes in the manner which reliably controls the size, size distribution and integrity of the particles in the initial dispersion. Liposomes which may be used in the invention may be MLVs, multivesicular liposomes (MVLs), large unilamellar vesicles (LUVs), and SUVs; and generally any microscopic particle having a lipid bilayer membrane. In the examples which follow, the reconstitution of dried SUVs having a particle size of less than 200nm is shown.

Techniques for the dehydration of cells and lipid bilayer vesicles in general, and liposomes in particular, are well known. The freeze drying of liposomes and other vesicles to form a freeze dried powder (a lyophilizate) can be conducted according to the method set forth in U.S. Patent 4,229,360; U.S. Patent 4,857,319; and copending U.S. application Serial No. 253,680 filed October 5, 1988. Liposomes may be dehydrated by spray drying or other methods of flash evaporation as described in published application WO 88/06441, and other dehydration methods may also be employed.

Generally with respect to lyophilization, the initial liposome dispersion, preferably containing a hydrophilic membrane stabilizing agent, is first frozen to a predetermined temperature, *e.g.,* -30°to -50°, and after the liquid mixture has solidified it is subjected to a reduced pressure, *(eg.,* 0.1 Torr) which causes the sublimation of the ice. The temperature and reduced pressure is then continued, as is known in the art, to remove residual moisture from the product, and the lyophilized powder thus obtained is then sealed and stored for use.

According to the invention, the vesicle powder thus obtained is reconstituted by adding a liquid aqueous phase under temperature conditions of less than room temperature, *ie.,* less than about 15°, to the dried liposome powder. Preferably, an aqueous phase is added at a temperature of less than 10°, and most preferably the liquid aqueous phase has a temperature of from about 2° to about 8°. The liquid aqueous phase may comprise any of a number of solutions, the composition of which is dictated by the particular liposome dispersion and the intended use thereof. For example, sterile water-for-injection (WFI) may be employed, as may 5% dextrose (D5W), phosphate buffered saline, or other solutions. The liquid aqueous phase may be placed in a refrigerator for a period of time sufficient to permit the liquid to attain the desired temperature, or in the case of liposomal formulations which are particularly sensitive to rehydration conditions the aqueous phase is drawn into a sterile syringe which is placed in a refrigerator until the required temperature is reached. In a clinical setting, following the infusion of the cold aqueous phase into the vesicle powder and the consequent rehydration of the vesicles, the temperature may be increased for further processing or injection as desired without damage to the size distribution of the vesicles.

In the example which follows, a dried liposome powder prepared from an initial dispersion of SUVs containing the antifungal agent amphotericin B is reconstituted by the method of the invention. The initial dispersion of liposomes was formed as described in patent publication EPA 0317120. Generally, a soluble complex was first formed between amphotericin B and distearoylphosphatidylglycerol in a solution of chloroform and methanol acidified to a pH of about 1.0 to 3.0. This amphotericin B/phospholipid complex was then mixed with phosphatidylcholine and cholesterol and dried to yield a lipid powder. The liposome preparation was accomplished by hydrating this lipid complex powder with an aqueous buffer having a pH of about 5.5 or less and including sucrose as a membrane stabilizing agent according to the teachings of U.S. Patent 4,857,319; and applying a shearing force such as sonication or homogenization to form the SUVs. In this instance, the liposomes were formed using the homogenizing apparatus described in U.S. Patent 4,753,788. The disclosures of both patents mentioned in this paragraph are incorporated herein by reference. The initial liposome dispersion, prior to dehydration, included amphotericin B-containing unilamellar liposomes of a mean diameter of less than 200nm, and having a narrow size distribution of particles as measured by the wavelength dependence of light scattering in a non-absorbing region of the visible spectrum (650 - 750nm), of approximately 4.0.

Eight lots of the initial amphotericin B liposome preparations were then filled into individual vials and lyophilized. Following lyophilization, the liposome powder in all vials was flushed with nitrogen and hermetically sealed.

The size distribution of particles in the reconstituted liposome dispersions set forth in Table I and the Figure was evaluated by measurement of the wavelength dependence of light scattering in a non-absorbing region of the visible spectrum (650 - 750nm), a technique described in Long, *et al., J. Colloid Interface Sci.* 42, 545 (1973), the disclosure of which is incorporated herein by reference. The negative slope of a plot of log (absorbance) vs. log (wavelength) for this spectral window is designated the scattering index (n). For dispersions of particles that are small relative to the wavelength of the scattered light, the index is approximately 4.0. The presence of aggregates or large particles is revealed by a decrease in scattering index to values that may be less than unity. While the releasability of pharmaceutical preparations is based on toxicity testing and a variety of other tests, an index value of about 3.0 or greater according to this modified Long *et al.* procedure correlates with superior acceptability, from a particle size standpoint, for parenteral use of the amphotericin B liposome preparations. An index value of about 2.5 or greater correlates with good acceptability, from a particle size standpoint, for such preparations. For the preparations of this example, the pre-lyophilization scattering index was approximately 3.9 to 4.1.

The liposome preparations in the vials were reconstituted by infusing WFI at selected temperatures. In column one of Table I the vessel containing WFI at 4° was removed from the refrigerator and an aliquot of WFI withdrawn into a syringe. The syringe was then placed in the refrigerator to equilibrate the WFI to 4° after exposure to room temperature before injection into the vial. Column two was estimated at 6° for 4° WFI which was removed from the refrigerator and injected into vials without equilibration. Columns three and four refer to the temperature of storage which was assumed not to change significantly. All other rehydrations were performed by removing a vessel containing WFI at the designated temperature from a controlled temperature environment and hydrating the liposome powder without equilibrating the WFI in the syringe. In clinics, all vials would be stored at 4°. All vials in the example were stored at room temperature prior to injection of the aqueous phase. Following the infusion of the cold water, the vials were each shaken vigorously for one to two minutes, and then heated to 65° for ten minutes prior to the evaluation of the size distribution of particles as described in the preceding paragraph.

The results show that rehydration below room temperature *(ie.,* less than 15°) provides good acceptability, from a particle size standpoint, for parenteral use of all of the tested lots of the amphotericin B liposome preparations. Rehydration at temperatures of 4°, 6° or 10° provided superior acceptability for all batches. In contrast, four lots were unacceptable, one was good and only three were superior at 20°, and five lots were unacceptable at the room temperature in a typical hospital or clinical environment, *i.e.,* about 26°.

The invention thus provides for the consistent and reliable reconstitution of dehydrated liposomal preparations through the use of a cold aqueous phase as a hydration medium. Particularly advantageous results are obtained in the reconstitution of lyophilized liposomal preparations, in which the low temperature overcomes the variability, due to other factors, in the reconstitution of similar batches of liposome product at room temperature.

TABLE I

WFI Temperature (Degrees Celsius)

| BATCH | 4 | 6 | 10 | 15 | 20 | 25 | 35 | 50 | 65 |
|---|---|---|---|---|---|---|---|---|---|
| 191S | 3.68 | 3.65 | 3.42 | 3.37 | 3.21 | 2.76 | 1.29 | | |
| 191V | 3.73 | 3.68 | 3.58 | 2.99 | 2.08 | 1.94 | 0.58 | | 0.38 |
| 189 | 3.49 | 3.40 | 3.32 | 2.77 | 2.32 | 1.53 | 0.62 | | |
| 175 | 3.41 | 3.12 | 2.94 | 2.62 | 2.80 | 1.92 | 0.97 | | |
| 168 | 3.33 | 3.25 | 3.21 | 2.70 | 2.24 | 1.83 | 1.18 | | |
| 156 | 3.69 | 3.74 | 3.68 | 3.72 | 3.60 | 3.56 | 3.47 | 3.23 | 2.72 |
| 187 | 3.63 | 3.41 | 3.01 | 2.84 | 2.25 | 1.51 | 0.96 | | |
| 270-055 | 3.58 | 3.73 | 3.72 | 3.63 | 3.44 | 3.16 | 2.02 | 1.08 | |

## Claims

1. A method for the reconstitution of dried lipid bilayer vesicles, comprising adding a quantity of a liquid aqueous phase having a temperature of less than 15°C to the dried vesicles to form a dispersion.

2. The method of claim 1 in which the vesicles are liposomes.

3. The method of claim 1 or 2 in which the vesicles are lyophilizates.

4. The method of claim 1, 2 or 3 in which the liquid aqueous phase has a temperature of less than 8°C.

5. The method of claim 1, 2, 3 or 4 in which the dried vesicles include a hydrophilic membrane stabilizing agent.

## Patentansprüche

1. Verfahren zur Rekonstitution von getrockneten Vesikeln mit Lipid-Doppelschicht, bestehend aus der Zugabe einer Menge einer flüssigen wässerigen Phase mit einer Temperatur von weniger als 15°C zu den getrockneten Vesikeln zur Bildung einer Dispersion.

2. Verfahren nach Anspruch 1, wobei die Vesikel Liposome sind.

3. Verfahren nach Anspruch 1 oder 2, wobei die Vesikel Lyophilisate sind.

4. Verfahren nach Anspruch 1, 2 oder 3, wobei die flüssige wässerige Phase eine Temperatur von weniger als 8°C aufweist.

5. Verfahren nach Anspruch 1, 2, 3 oder 4, wobei die getrockneten Vesikel ein Stabilisierungsmittel der hydrophilen Membran enthalten.

## Revendications

1. Procédé de reconstitution de vésicules lipidiques séchées à deux couches, qui consiste à ajouter une certaine quantité d'une phase aqueuse liquide ayant une température inférieure à 15°C aux vésicules séchées pour former une dispersion.

2. Procédé suivant la revendication 1, dans lequel les vésicules sont des liposomes.

3. Procédé suivant la revendication 1 ou 2, dans lequel les vésicules sont des lyophilisats.

4. Procédé suivant la revendication 1, 2 ou 3, dans lequel la phase liquide aqueuse a une température inférieure à 8°C.

5. Procédé suivant la revendication 1, 2, 3 ou 4, dans lequel les vésicules séchées comprennent un agent hydrophile de stabilisation de membrane.

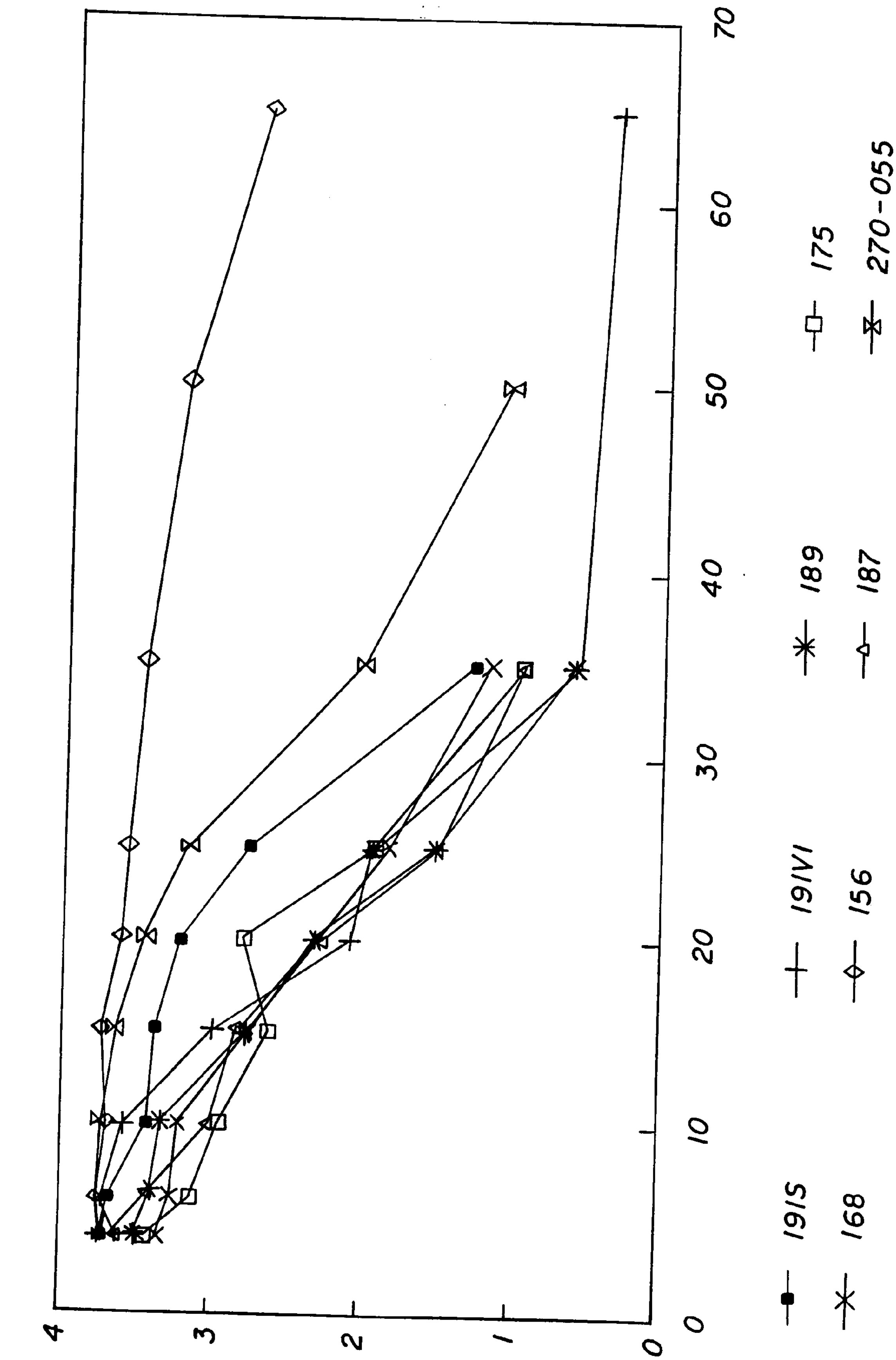
0
1
2
3
4
0
10
20
30
40
50
60
70
191S
168
191VI
156
189
187
175
270-055